# EUROPEAN PATENT SPECIFICATION

(11) **EP 1 266 618 B1**
(45) Date of publication and mention of the grant of the patent: **14.12.2005**
(21) Application number: 01113514.2
(22) Date of filing: 11.06.2001
(51) Int. Cl.: A61B 5/15

(54) **Safety vacuum syringe for blood sampling conformed to ergonomics**
Ergonomisch gestaltete Sicherheitsvakuumspritze für Blutproben
Seringue de sécurité à forme ergonomique pour prise de sang

(43) Date of publication of application: 18.12.2002
(60) Divisional application: 04000220.6
(73) Proprietor: Chen, Long-Hsiung, Taipei (TW)
(72) Inventor: Chen, Long-Hsiung, Taipei (TW)
(74) Representative: Casalonga, Axel

(56) References cited:
- EP-A- 0 489 977
- EP-A- 0 908 193
- WO-A-92/04867
- US-A- 5 897 508
- US-A- 6 152 901

## Description

The present invention relates to a safety vacuum syringe for blood sampling conformed to ergonomics, and more particularly to the syringe that not only conforms to ergonomics in use but may receive the conventional needle head and the vacuum tube so as to lower production cost.

Many viruses and diseases or bodily functions can be examined by a blood test, which makes the blood test to be a regular and frequent job. In preventing a patient from infecting lethal virus or germ, the selection of blood sampling tool will be very important during a blood sampling process. Therefore, in order to make sampling safer, blood sampling must have some degree of improvement.

The needle head of a conventional blood sampling syringe, as revealed in U.S. Patent 5,423,758, is disposed at the central axis of the front end of the syringe. During a blood sampling process, the needle of a syringe is stuck into a patient's vein to draw blood from the vein. But the angle between the syringe and the skin surface of a human body is large when blood sampling is processed because the needle head is at the center of syringe. The needle head is difficult to be inserted into the vein to draw blood. This is specially true for the patients whose blood vessels are thinner because it is easier for the needle to pass through the blood vessel. Thus, blood sampling cannot be done smoothly.

The U.S. patent 6,152,901 discloses a blood sampling device comprising a tubular body including a side wall provided with at least one longitudinal slot ending in rear and front enlargements, and a front transverse wall provided with an orifice, and a cannula mounted on a slide. The slide is displaceable in the tubular body between a rear end position in which the front part of the cannula are situated inside the tubular body and a front end position in which the front part of the cannula passes through the orifice in the transverse wall.

One objective of the present invention is to provide a savety vacuum syringe for blood sampling conformed to ergonomics, whereby a needle head is eccentrically disposed at the front end of a syringe to lower the blood sampling angle during the blood sampling process allowing blood sampling to be done smoothly.

Another objective of the present invention is to provide a safety vacuum syringe for blood sampling, wherein an inner needle tip of the needle head has a step shape comprising two right angles so that it can be positioned at the center of the needle head to fit the traditional vacuum blood collecting cup.

Still another objective of the present invention is to provide a conventional needle head that can be used according to a users' need.

According to a first embodiment as claimed, a safety vacuum syringe for blood sampling conformed to ergonomics, mainly comprises a hollow barrel, a reduced lining tube and a vacuum tube. The hollow barrel has a reduced inlet disposed on the top end thereof. The reduced lining tube is installed in said hollow barrel, an eccentric reduced portion being eccentrically disposed on the top end of said reduced lining tube and having an opening at the lower end thereof. Said eccentric reduced portion being wedged and fixed in said reduced lining tube. Furthermore, a through guiding hole is disposed at the center of said eccentric reduced portion, and said hollow barred comprises a guiding slit disposed at the flank side thereof. A press plate is connected to said reduced lining tube at the outer flank side thereof and being positioned in said guiding slit.

According to the invention, the eccentric reduced portion projects out of the reduced inlet and a needle head is fixedly covered on said eccentric reduced portion. Furthermore, a step shaped needle comprising two right angles is positioned in said through guiding hole, and a lower needle tip of said needle is positioned at the central axis in said reduced lining tube.

According to a second embodiment as claimed, a safety vacuum syringe for blood sampling conformed to ergonomics, mainly comprises a hollow barrel, a reduced lining tube and a vacuum tube. The hollow barrel has a reduced inlet disposed on the top end thereof. The reduced lining tube is installed in said hollow barrel, an eccentric reduced portion being eccentrically disposed on the top end of said reduced lining tube and having an opening at the lower end thereof. Said eccentric reduced portion being wedged and fixed in said reduced lining tube. Furthermore, a through guiding hole is disposed at the center of said eccentric reduced portion, and said hollow barred comprises a guiding slit disposed at the flank side thereof. A press plate is connected to said reduced lining tube at the outer flank side thereof and being positioned in said guiding slit.

According to the invention, the eccentric reduced portion projects out of the reduced inlet and a needle cannula is located in said through guiding hole. Furthermore, a step shaped needle comprising two right angles is positioned in said through guiding hole, and a lower needle tip of said needle being positioned at the central axis in said reduced lining tube.

The present invention can be better understood by detailed description of the following drawings, in which:
FIG. 1 is a longitudinal sectional view of a preferred embodiment of the present invention before a needle holder is mounted on a syringe;
FIG. 2 is a longitudinal sectional view of a preferred embodiment of the present invention after a needle holder is mounted on a syringe;
FIG. 3 is a side view of a preferred embodiment of the present invention, showing a guide groove.
FIG. 4 is a longitudinal sectional view of a preferred embodiment of the present invention, showing the structure inside;
FIG. 4A is a sectional view of a preferred embodiment of the present invention when viewed along the section line of FIG 4;
FIG. 5 is an explosive view of a reduced lining tube;
FIG. 6 is a longitudinal sectional view of a preferred embodiment of the present invention during use;
FIG. 7 is a longitudinal sectional view of a preferred embodiment of the present invention, showing a condition withdrawing a reduced lining tube back;
FIG. 8 is a longitudinal sectional view of a preferred embodiment of the present invention, showing a needle head being directly inserted into an eccentric reduced portion;
FIG. 9 is a longitudinal sectional view of another example of the present invention, showing a press plate being separated from a syringe;
FIG. 10 is a longitudinal sectional view of another example of the present invention, showing a press plate being wedged into a syringe;
FIG. 11 is an explosive view of the front part of another example of the present invention;
FIG. 12 is a perspective view of the front part of another example of the present invention;
FIG. 13 is a longitudinal sectional view of another example of the present invention during use;
FIG. 14 is a schematic longitudinal sectional view of another example of the present invention, showing a needle seat being separated from a syringe; and
FIG. 15 is a schematic longitudinal sectional view of another example of the present invention, showing a needle seat being wedged into a syringe.

### DETAILED DESCRIPTIONS OF THE INVENTION

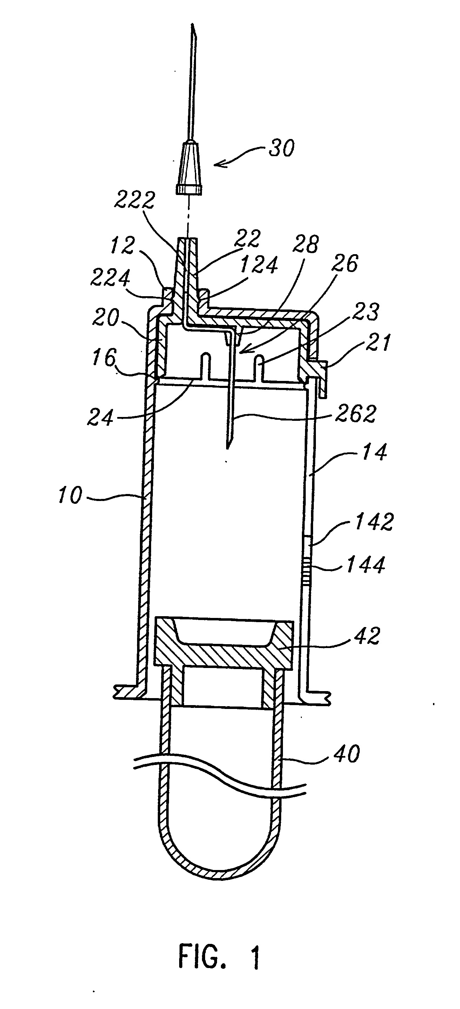

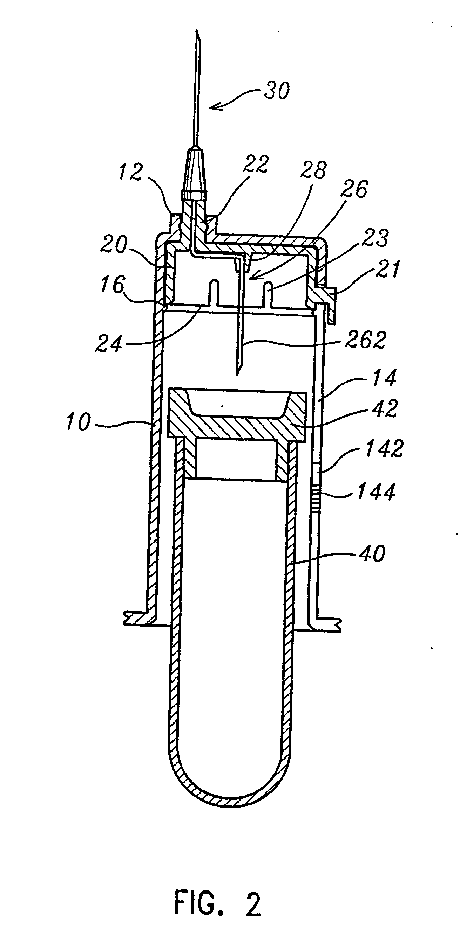

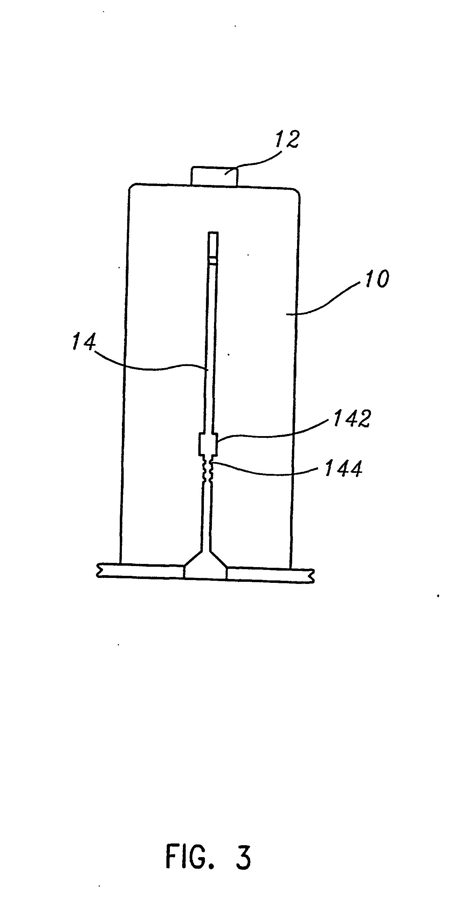

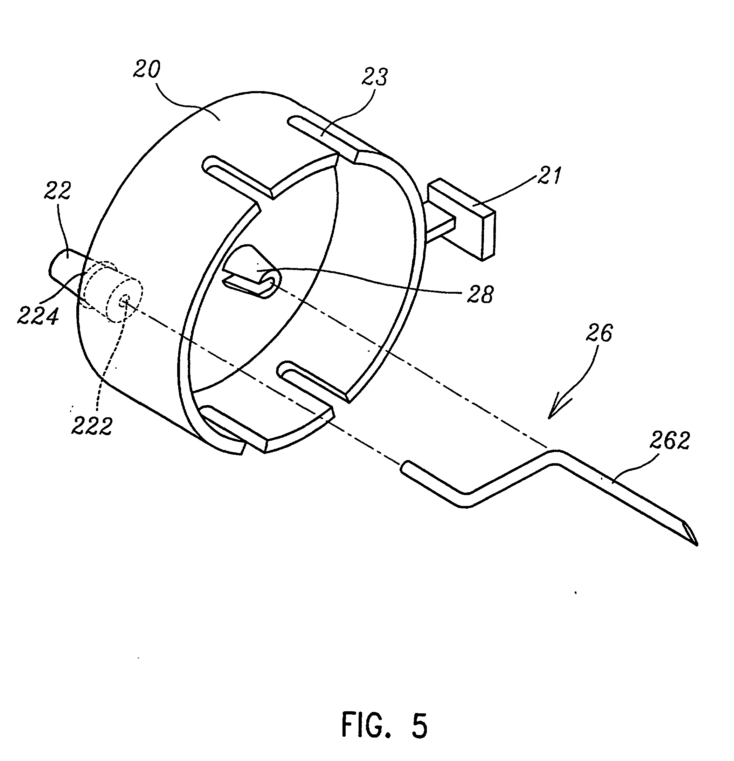

As shown in FIG. 1, 2 and 3, the present invention comprises a hollow barrel 10 a reduced inlet 12 eccentrically disposed at the top end of the barrel 10 and a guide slit 14 at the flank side of the barrel 10. The barrel comprises a reduced lining tube 20, an eccentric reduced portion 22 is disposed at a position away from the center of the top end of the reduced lining tube 20 and an opening 24 is disposed at the bottom thereof. The eccentric reduced portion 22 can be embedded into the reduced inlet 12 and projects out of the reduced inlet 12. A needle head 30 can be wedged onto the eccentric reduced portion 22, and a through guiding hole 222 is disposed at the center of the eccentric reduced portion 22 so that a step shaped needle 26 comprising two right angles can be fixed therein and a lower needle tip 262 of the needle 26 can be placed at the central axial line of the reduced lining tube 20. A fixing seat 28 is disposed at the center of the inner section of the reduced lining tube 20 for receiving and fixing the needle 26 therein. As shown in FIG. 5, a press plate 21 is further attached to the reduced lining tube 20 at the outer surface thereof and positioned in the guiding slit 14 to be utilized to drive the reduced lining tube to move in the longitudinal direction of the barrel 10.
A hook-shaped flange 16 is further disposed at a proper position of the inner surface of the barrel 10 so as to position the reduced lining tube 20 at the top end of the barrel 10 and not to slide loosely therein. Furthermore, at least one circular flange 224 is disposed around the eccentric reduced portion 22 and at least one circular groove 124 is disposed around the inner surface of the reduced inlet 12 to accommodate and position the circular groove 124. A plurality of small slits 23 are cut around lower side of the reduced lining tube 20, enabling the lower end of the reduced lining tube to maintain its proper elasticity. A vacuum tube 40 is further disposed inside the barrel 10, and an elastic plug 42 is fixedly covered in an opening that is at the top end of the vacuum tube 40. The vacuum tube 40 is convertibly available.

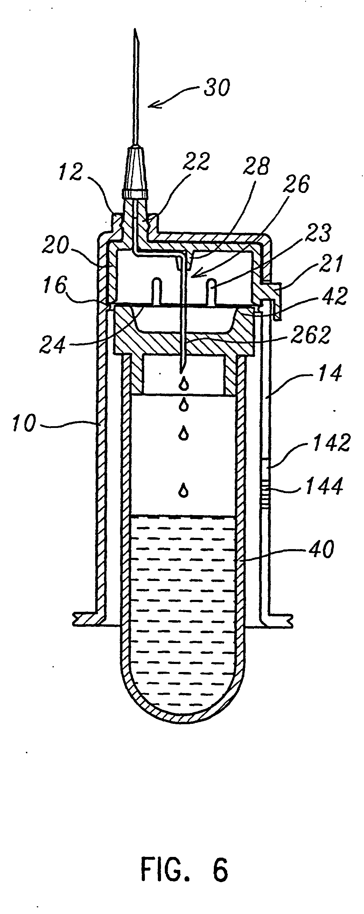

Please refer to FIG. 6, when processing a blood sampling; the needle head 30 is inserted into the vein of a patient, and the vacuum tube 40 moves toward the needle 26 inside the barrel 10 in order to force the lower needle tip 262 to prick through the plug 42 on the top of the cup 40. The blood from the vein of the patient will pass through the needle head 30, the guiding hole 222, the needle 26, and into the vacuum blood collecting cup 40. Since the needle head 30, the eccentric reduced portion 22 and reduced inlet 12 are all eccentrically disposed and close to the circumference of the barrel 10, a health care worker can prick the needle head 30 into the vein with a smaller inclined angle to the skin of the patient. Thus, the vaccuum syringe not only conforms to ergonomics, but prevents the needle head piercing through the blood vessels.

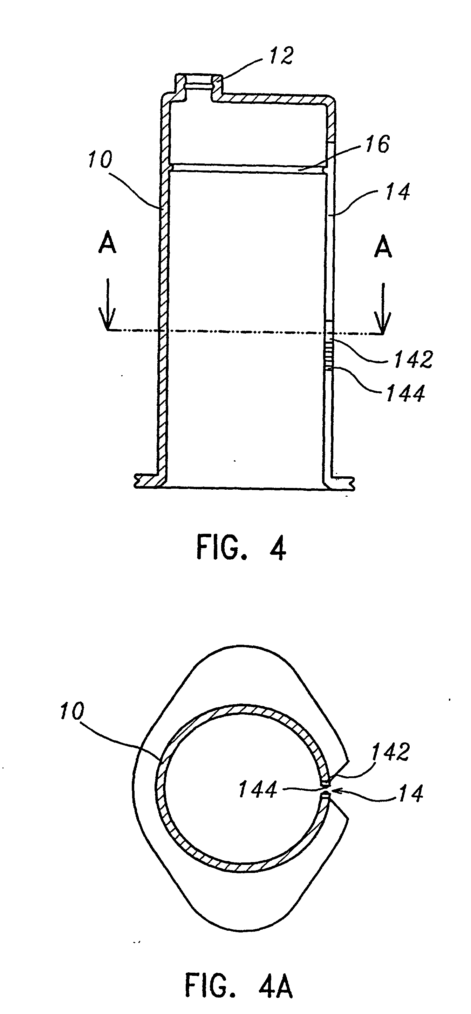

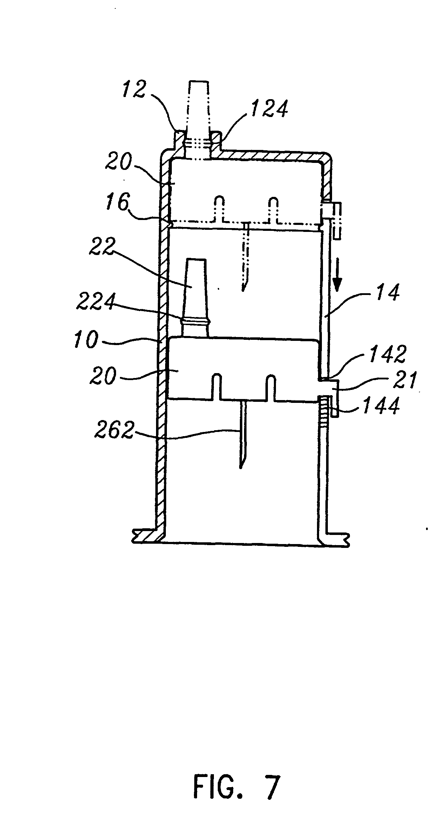

Referring to FIG. 7, after the blood sampling is over, the press plate is pressed 21 backward to move the reduced lining tube back to prevent the needle head from pricking the health care workers. A stopping groove 142 is disposed at a proper position of the guiding slit 14, the width of the stopping groove 142 is larger than the width of the guiding slit 14 in order to stop the press plate 21 to moving forward or backward when the press plate 21 is moved to the stopping groove 142 to avoid the lower needle tip 262 at the lower end of the needle 26 to project out of the barrel 10 to cause danger. A plurality of bulging points 144 are disposed at the section of the guiding slit 14 below the stopping groove 142 to prevent the press plate 21 from slipping backward to drop out of the stopping groove 142 resulting in the lower needle tip being exposed from the barrel 10, as shown in FIG. 4 and 4A.

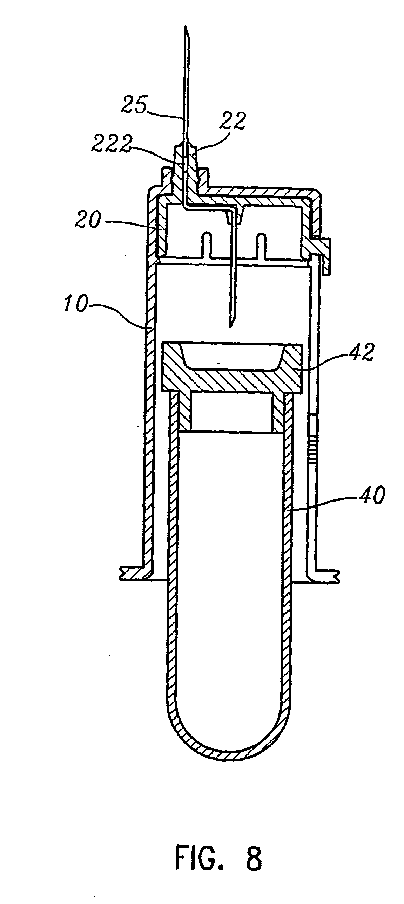

Referring also to FIG. 8, a cannula needle can be located in the guiding hole 222 in advance when the eccentric reduced portion 22 of the reduced lining tube 20 is in production. Thus, there is no need to insert the needle separately, saving time and production cost.

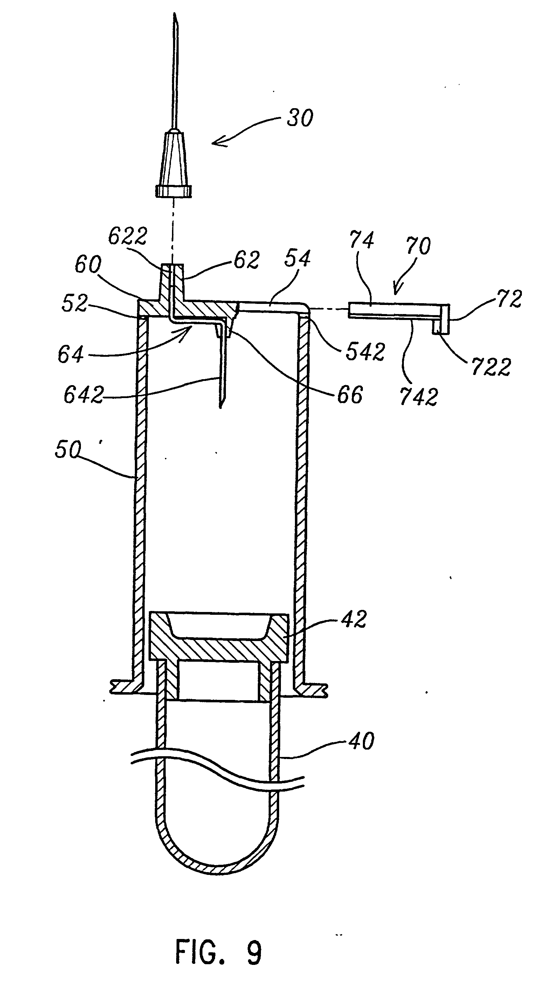

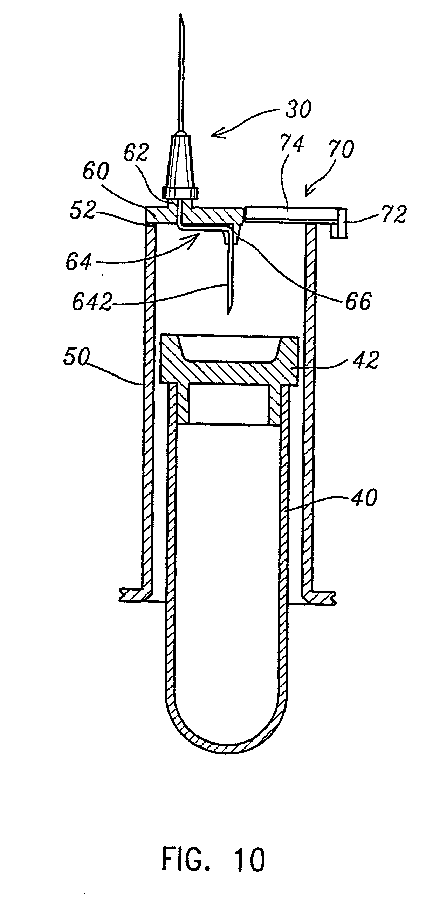

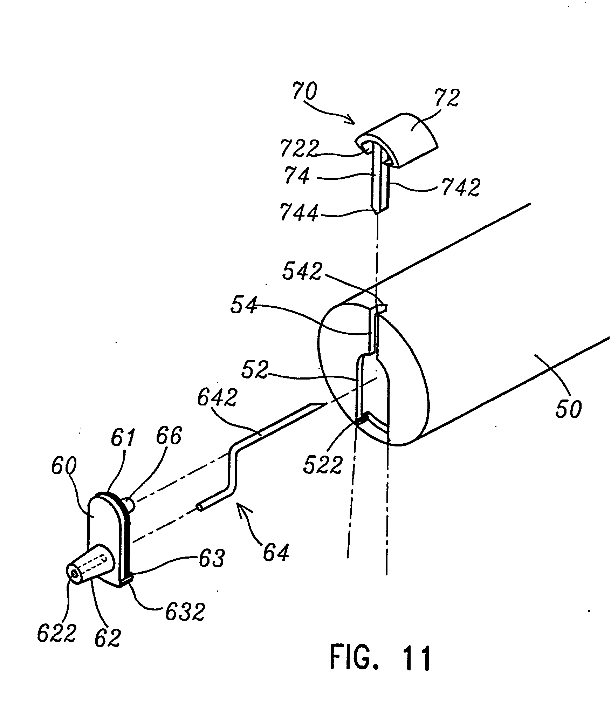

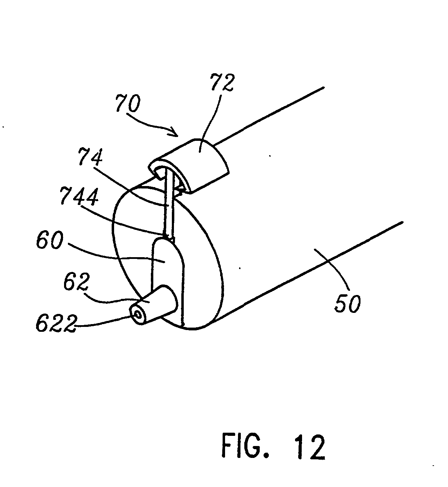

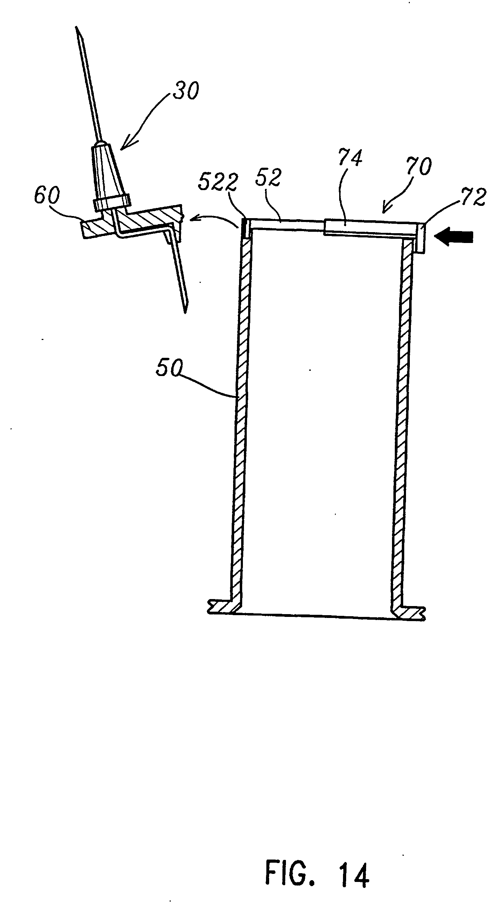

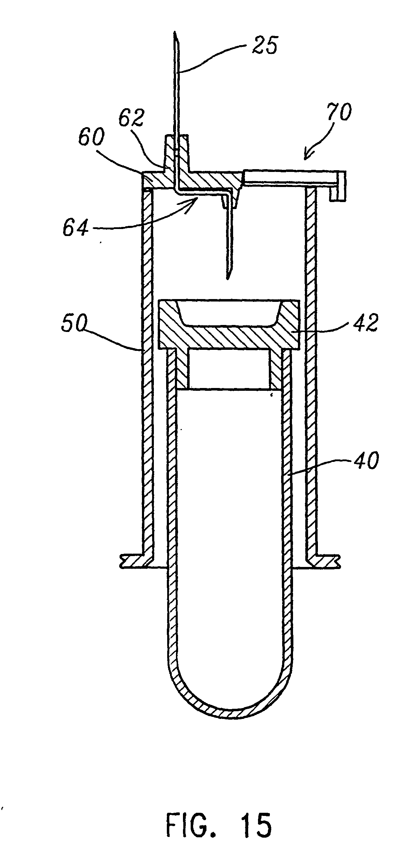

FIGS. 9-15 show further examples comprising a hollow barrel 50, a directional sliding trench 52 is transversely disposed at one side of the front end of the barrel 50, and a sliding slit 54 transversely disposed at another side opposite to the trench. A needle seat 60 and a press plate 70 are further disposed in the trench 52 and the slit 54 respectively. An eccentric reduced portion 62 is still disposed on the upper surface of the needle seat, and a needle head 30 may be wedged on the eccentric reduced portion 62. A guiding hole is pierced through the eccentric reduced port ion 62 so that a step shaped needle 64 comprising two night angles can be fixed therein and a lower needle tip 642 of the needle 64 can be placed at the central axial line of the reduced lining tube 50 firmly. A fixing seat 66 is disposed at the lower side of the needle seat 20 for receiving and fixing the needle 64 therein. Because the needle seat 60 can be fixed firmly in the trench 52, a positioning flange 61 is disposed around the outside profile of the needle seat 60, and a small flange 632 is attached to each flank side of a convex plate 63. A guiding groove 522 is disposed at each flank side of the trench corresponding to the position of the flange 632 so as to fix the flange therein to prevent the needle seat from dropping out of the trench. Moreover, in order to assemble the needle seat 60 easily into the trench 52, the trench 52 has a certain degree of angle between its two sides so that the width of the opening at the front edge is larger than the one of the end, enabling the needle seat 60 to easily slip into the trench 52. The press plate 70 has an end face 72 and a guiding stick 74, the guiding stick 74 has a triangle shape strip 742 at its flank side. The sliding slit 54 has a triangle shape slit opening 542 at its flank side corresponding to the position of the strip 742, so as to move the press plate 70 as a guide. A triangle shape inverted hook is formed at the end of the guiding stick 74 in order to be connected with the joint of the slit 54 and the trench 52 when the press plate 70 is positioned in the slit 54, enabling the press plate 70 not to slide backward to separate from the slit 54. An arc spring plate 722 is connected to the end of each flank side below the end face 72 to provide proper elastic force. A vacuum tube 40 is further disposed in the barrel 50, and an elastic plug 42 is firmly covered in the opening at the upper end of the vacuum tube 40, wherein the vacuum tube is available in the market.

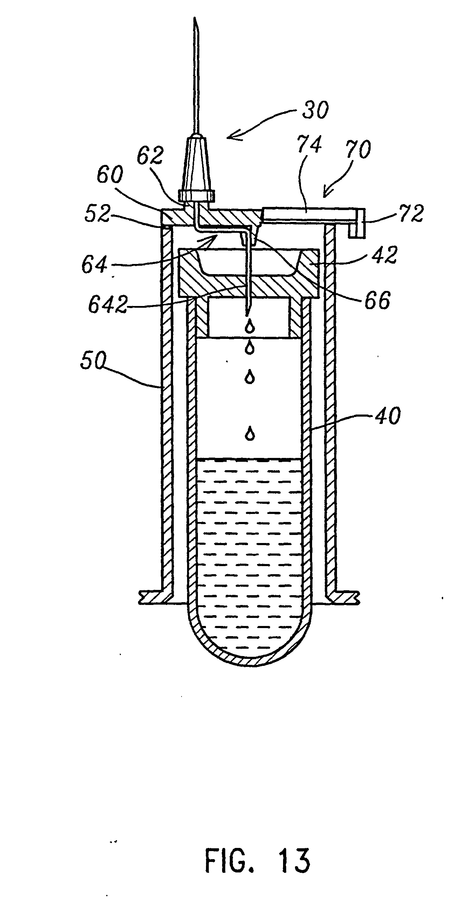

Next, referring to FIG. 13, when taking sample a blood, strike the needle head into the vein of a patient, then move the vacuum tube 40 toward the needle 64 inside the barrel 10 to allow the plug 42 of the cup 40 to be pierced. The blood from the vein of the patient will pass through the needle head 30, the guiding hole 622, to the needle 64, and into the vacuum blood collecting cup 40. When the blood sampling is over, the press plate 70 may be pushed to separate the needle seat 60 from the trench, and then to throw it in a needle head collector. Therefore, change the needle seat 60 and needle head for the next blood sampling to avoid the infection of virus.

The advantage of the invention is that a proper thickness needle head 30 may be chosen to wedge on the eccentric reduced portion 22, 62, and the needle head can be a conventional needle head instead of using a particular one that needs a new mold to produce saving costs. Moreover, since a conventional vacuum blood collecting cup can still be used after the needle head 30 is eccentrically disposed, the needle 26, 64 of the invention is designed to be like a step comprising two right angle so as to position the lower needle tip 262, 642 of the needle 26, 64 at the center interior the barrel 10 and 50. Therefore, the lower needle tip 262, 642 can prick through the plug at the center, alleviating the need for vacuum blood collecting cup is unnecessary.

It is to be understood that the drawing is designed for purpose of illustration only, and is not intended for use as a definition of the limits and scope of the invention disclosed.

## Claims

1. A safety vacuum syringe for blood sampling conformed to ergonomics, mainly comprising a hollow barrel (10), a reduced lining tube (20) and a vacuum tube (40);
the hollow barrel (10) having a reduced inlet (12) disposed on the top end thereof;
the reduced lining tube (20) being installed in said hollow barrel (10); an eccentric reduced portion (22) being eccentrically disposed on the top end of said reduced lining tube (20) and having an opening (24) at the lower end thereof;
said eccentric reduced portion (22) being wedged and fixed in said reduced lining tube (20);
a through guiding hole (222) being disposed at the center of said eccentric reduced portion (22);
said hollow barrel (10) comprising a guiding slit (14) disposed at the flank side thereof; a press plate (21) being connected to said reduced lining tube (20) at the outer flank side thereof and being positioned in said guiding slit (14),
**characterized in that**:
the eccentric reduced portion (22) projects out of the reduced inlet (12);
a needle head (30) being fixedly covered on said eccentric reduced portion (22);
a step shaped needle (26) comprising two right angles being positioned in said through guiding hole (222);
a lower needle tip (262) of said needle step shaped (26) being positioned at the central axis in said reduced lining tube (20).

2. The syringe of claim 1, wherein in order to install said lower needle tip at the center of said reduced lining tube, a fixing seat being disposed at the center inside said reduced lining tub for said needle to be fixed therein.

3. The syringe of claim 1, wherein a hook-shaped flange (16) is disposed around inner wall of said barrel at a proper position so as to fix said reduce lining tube at the top end of said barrel and not to slip loosely downward.

4. The syringe of claim 1, wherein a plurality of small slits (23) are disposed around lower side of said reduced lining tube, enabling the lower end of said reduced lining tube to keep proper elasticity.

5. The syringe of claim 1, wherein a circular flange (224) is disposed around the eccentric reduced portion and a circular groove is disposed around the inner surface of said reduced inlet to accommodate and position said eccentric reduced portion in said reduced inlet.

6. The syringe of claim 1, wherein a stopping groove (142) is disposed at a proper position of said guiding slit, the width of said stopping groove is larger than the width of said guiding slit.

7. The syringe of claim 6, wherein a plurality of bulging points (144) are disposed at the section of the guiding slit below the stopping groove.

8. A safety vacuum syringe for blood sampling conformed to ergonomics, mainly comprising a hollow barrel (10), a reduced lining tube (20) and a vacuum tube (40);
the hollow barrel having a reduced inlet (12) disposed on the top end thereof;
the reduced lining tube (20) being installed in said hollow barrel;
an eccentric reduced portion (22) being eccentrically disposed on the top end of said reduced lining tube and having an opening (24) at the lower end thereof;
said eccentric reduced portion being wedged and fixed in said reduced lining tube;
a through guiding hole (222) being disposed at the center of said eccentric reduced portion (22);
said hollow barrel (10) comprising a guiding slit (14) disposed at the flank side thereof; a press plate (21) being connected to said reduced lining tube (20) at the outer flank side thereof and being positioned in said guiding slit (14);
**characterized in that**:
the eccentric reduced portion (22) projects out of the reduced inlet (12);
a needle cannula (25) being located in said through guiding hole (222);
a step shaped needle (26) comprising two right angles being positioned in said through guiding hole (222);
a lower needle tip (262) of said needle step shaped (26) being positioned at the central axis in said reduced lining tube (20).

## Patentansprüche

1. Ergonomisch gestaltete Sicherheitsvakuumspritze für Blutproben, im Wesentlichen umfassend einen hohlen, einseitig abgeschlossenen Zylinder (10), ein kleiner ausgelegtes Auskleidungsrohr (20) und ein Vakuumrohr (40);
wobei der hohle, einseitig abgeschlossene Zylinder (10) einen verengten Einlass (12) aufweist, der an seinem oberen Ende angeordnet ist;
wobei das kleiner ausgelegte Auskleidungsrohr (20) in dem hohlen, einseitig abgeschlossenen Zylinder (10) eingesetzt ist; wobei ein exzentrischer, verengter Abschnitt (22) am oberen Ende des kleiner ausgelegten Auskleidungsrohrs (20) angeordnet ist und eine Öffnung (24) an seinem unteren Ende aufweist;
wobei der exzentrische, verengte Abschnitt (22) in dem kleiner ausgelegten Auskleidungsrohr (20) verkeilt und befestigt ist;
wobei ein Durchgangsführungsloch (222) in der Mitte des exzentrischen, verengten Abschnitts (22) angeordnet ist;
wobei der hohle, einseitig abgeschlossene Zylinder (10) einen Führungsschlitz (14) umfasst, welcher an einer Flankenseite desselben angeordnet ist; wobei eine Druckplatte (21) mit dem kleiner ausgelegten Auskleidungsrohr (20) an dessen äußerer Flankenseite verbunden und in dem Führungsschlitz (14) positioniert ist,
**dadurch gekennzeichnet, dass**:
der exzentrische, verengte Abschnitt (22) aus dem verengten Einlass (12) hinausragt;
ein Nadelkopf (30) fest auf dem exzentrischen, verengten Abschnitt (22) abgedeckt ist;
eine gestuft geformte Nadel (26), welche zwei rechte Winkel umfasst, in dem Durchgangsführungsloch (222) positioniert ist;
eine untere Nadelspitze (262) der gestuft geformten Nadel (26) an der Mittelachse in dem kleiner ausgelegten Auskleidungsrohr (20) positioniert ist.

2. Spritze gemäß Anspruch 1, wobei, um die untere Nadelspitze an der Mittelachse in dem kleiner ausgelegten Auskleidungsrohr einzubauen, ein Befestigungsansatz an der Mittelachse in dem kleiner ausgelegten Auskleidungsrohr angeordnet ist, um die Nadel darin zu befestigen.

3. Spritze gemäß Anspruch 1, wobei ein hakenförmiger Flansch (16) um die innere Wand des einseitig abgeschlossenen Zylinders an einer geeigneten Position angeordnet ist, um so das kleiner ausgelegte Auskleidungsrohr am oberen Ende des einseitig abgeschlossenen Zylinders zu befestigen und es nicht lose nach unten rutschen zu lassen.

4. Spritze gemäß Anspruch 1, wobei eine Mehrzahl von kleinen Schlitzen (23) um die untere Seite des kleiner ausgelegten Auskleidungsrohrs angeordnet sind, um das untere Ende des kleiner ausgelegten Auskleidungsrohrs die geeignete Elastizität beibehalten zu lassen.

5. Spritze gemäß Anspruch 1, wobei ein kreisförmiger Flansch (224) um den exzentrischen, verengten Abschnitt angeordnet ist und eine kreisförmige Nut um die innere Oberfläche des verengten Einlasses angeordnet ist, um den exzentrischen, verengten Abschnitt im verengten Einlass aufzunehmen und zu positionieren.

6. Spritze gemäß Anspruch 1, wobei eine Anschlagnut (142) an einer geeigneten Position des Führungsschlitzes angeordnet ist, wobei die Breite der Anschlagnut größer als die Breite des Führungsschlitzes ist.

7. Spritze gemäß Anspruch 6, wobei eine Mehrzahl von ausgebauchten Punkten (144) an einem Abschnitt des Führungsschlitzes unter der Anschlagnut angeordnet ist.

8. Ergonomisch gestaltete Sicherheitsvakuumspritze für Blutproben, im Wesentlichen umfassend einen hohlen, einseitig abgeschlossenen Zylinder (10), ein kleiner ausgelegtes Auskleidungsrohr (20) und ein Vakuumrohr (40);
wobei der hohle, einseitig abgeschlossene Zylinder einen verengten Einlass (12) aufweist, der an seinem oberen Ende angeordnet ist;
wobei das kleiner ausgelegte Auskleidungsrohr (20) in dem hohlen, einseitig abgeschlossenen Zylinder eingesetzt ist;
wobei ein exzentrischer, verengter Abschnitt (22) am oberen Ende des kleiner ausgelegten Auskleidungsrohrs angeordnet ist und eine Öffnung (24) an seinem unteren Ende aufweist;
wobei der exzentrische, verengte Abschnitt in dem kleiner ausgelegten Auskleidungsrohr verkeilt und befestigt ist;
wobei ein Durchgangsführungsloch (222) in der Mitte des exzentrischen, verengten Abschnitts (22) angeordnet ist;
wobei der hohle, einseitig abgeschlossene Zylinder (10) einen Führungsschlitz (14) umfasst, welcher an einer Flankenseite desselben angeordnet ist; wobei eine Druckplatte (21) mit dem kleiner ausgelegten Auskleidungsrohr (20) an dessen äußerer Flankenseite verbunden und in dem Führungsschlitz (14) positioniert ist,
**dadurch gekennzeichnet, dass**:
der exzentrische, verengte Abschnitt (22) aus dem verengten Einlass (12) hinausragt;
eine Nadelkanüle (25) im Durchgangsführungsloch (222) angeordnet ist;
eine gestuft geformte Nadel (26), welche zwei rechte Winkel umfasst, in dem Durchgangsführungsloch (222) positioniert ist;
eine untere Nadelspitze (262) der gestuft geformten Nadel (26) an der Mittelachse in dem kleiner ausgelegten Auskleidungsrohr (20) positioniert ist.

## Revendications

1. Seringue de sécurité de forme ergonomique pour prise de sang, comprenant principalement un cylindre creux (10), un tube de doublage réduit (20) et un tube d'aspiration (40) ;
le cylindre creux (10) ayant un orifice d'entrée réduit (12) disposé sur son extrémité supérieure ;
le tube de doublage réduit (20) étant installé dans ledit cylindre creux (10) ;
une partie réduite excentrée (22) étant disposée de manière excentrée sur l'extrémité supérieure dudit tube de doublage réduit (20) et comportant une ouverture (24) en son extrémité inférieure ;
ladite partie réduite excentrée (22) comportant une saillie et étant fixée dans ledit tube de doublage réduit (20) ;
un trou de guidage traversant (222) étant pratiqué au centre de ladite partie réduite excentrée (22) ;
le cylindre creux (10) comprenant une fente de guidage (14) pratiquée sur le côté latéral de celui-ci ;
une plaquette de pressage (21) étant connectée audit tube de doublage réduit (20) sur le côté latéral extérieur de ce dernier et positionnée dans ladite fente de guidage (14),
**caractérisée en ce que** :
la partie réduite excentrée (22) fait saillie hors de l'orifice d'entrée réduit (12) ;
une tête à aiguille (30) recouvrant de manière fixe ladite partie réduite excentrée (22) ;
une aiguille en zigzag (26) comprenant deux angles droits étant positionnée dans ledit trou de guidage traversant (222) ;
une extrémité inférieure d'aiguille (262) de ladite aiguille en zigzag (26) étant positionnée sur l'axe central dudit tube de doublage réduit (20).

2. Seringue selon la revendication 1, dans laquelle, afin d'installer ladite extrémité inférieure d'aiguille au centre dudit tube de doublage réduit, un siège de fixation est prévu au centre, à l'intérieur dudit tube de doublage réduit, pour pouvoir y fixer ladite aiguille.

3. Seringue selon la revendication 1, dans laquelle un rebord d'accrochage (16) est prévu autour de la paroi intérieure dudit cylindre en une position qui permet de fixer ledit tube de doublage réduit à l'extrémité supérieure dudit cylindre et qui permet à ce même tube de doublage de ne pas glisser vers le bas.

4. Seringue selon la revendication 1, dans laquelle une pluralité de petites fentes (23) sont pratiquées autour du côté inférieur dudit tube de doublage réduit, permettant à l'extrémité inférieure dudit tube de doublage réduit de conserver une élasticité correcte.

5. Seringue selon la revendication 1, dans laquelle un rebord circulaire (224) est disposé autour de la partie réduite excentrée et une rainure circulaire est formée autour de la surface intérieure dudit orifice d'entrée réduit pour loger et positionner ladite partie réduite excentrée dans ledit orifice d'entrée réduit.

6. Seringue selon la revendication 1, dans laquelle une rainure d'arrêt (142) est placée en une bonne position de ladite fente de guidage, la largeur de ladite rainure d'arrêt étant supérieure à la largeur de ladite fente de guidage.

7. Seringue selon la revendication 6, dans laquelle une pluralité de points saillants (144) sont disposés au niveau de la portion de la fente de guidage qui se trouve sous la rainure d'arrêt.

8. Seringue de sécurité de forme ergonomique pour prise de sang, comprenant principalement un cylindre creux (10), un tube de doublage réduit (20) et un tube d'aspiration (40) ;
le cylindre creux ayant un orifice d'entrée réduit (12) disposé sur son extrémité supérieure ;
le tube de doublage réduit (20) étant installé dans ledit cylindre creux (10) ;
une partie réduite excentrée (22) étant disposée de manière excentrée sur l'extrémité supérieure dudit tube de doublage réduit et comportant une ouverture (24) en son extrémité inférieure ;
ladite partie réduite excentrée comportant une saillie et étant fixée dans ledit tube de doublage réduit ;
un trou de guidage traversant (222) étant pratiqué au centre de ladite partie réduite excentrée (22) ;
ledit cylindre creux (10) comprenant une fente de guidage (14) pratiquée sur le côté latéral de celui-ci ;
une plaquette de pressage (21) étant connectée audit tube de doublage réduit (20) sur le côté latéral extérieur de ce dernier et positionnée dans ladite fente de guidage (14),
**caractérisée en ce que** :
la partie réduite excentrée (22) fait saillie hors de l'orifice d'entrée réduit (12) ;
une canule en forme d'aiguille (25) étant placée dans ledit trou de guidage traversant (222) ;
une aiguille en zigzag (26) comprenant deux angles droits étant positionnée dans ledit trou de guidage traversant (222) ;
une extrémité inférieure d'aiguille (262) de ladite aiguille en zigzag (26) étant positionnée sur l'axe central dudit tube de doublage réduit (20).
